# EUROPEAN PATENT SPECIFICATION

(11) **EP 1 993 622 B1**
(45) Date of publication and mention of the grant of the patent: **29.07.2009**
(21) Application number: 07702535.1
(22) Date of filing: 05.03.2007
(51) Int. Cl.: A61L 15/44

(54) **A WOUND DRESSING COMPRISING AN ANTI-INFLAMMATORY PAIN-KILLING AGENT AND A COMPLEX OF SILVER ION AND A TRANSITIONAL ELEMENT OF GROUP IV OF THE PERIODIC SYSTEM OF ELEMENTS**
WUNDVERBAND MIT EINEM ENTZÜNDUNGSHEMMENDEN SCHMERZMITTEL UND EINEM KOMPLEX AUS SILBERION UND EINEM ÜBERGANGSELEMENT DER GRUPPE IV DES PERIODENSYSTEMS DER ELEMENTE
PANSEMENT DE PLAIE COMPRENANT UN AGENT ANALGÉSIQUE ANTI-INFLAMMATOIRE ET UN COMPLEXE D'ION ARGENT ET UN ÉLÉMENT TRANSITIONNEL DU GROUPE IV DU SYSTÈME PÉRIODIQUE DES ÉLÉMENTS

(30) Priority: 03.03.2006 DK 200600310; 03.03.2006 DK 200600311
(43) Date of publication of application: 26.11.2008
(73) Proprietor: Coloplast A/S, 3050 Humlebæk (DK)
(72) Inventor: QVIST, Michael H., DK-3480 Fredenborg (DK); LAUEMOELLER, Sanne Lise, DK-3200 Helsinge (DK)
(86) International application number: PCT/DK2007/050027
(87) International publication number: WO 2007/098772

(56) References cited:
- WO-A-02/062403
- WO-A-03/055536
- WO-A-20/06018026
- WO-A-20/06089551
- WO-A2-02/078755
- GB-A- 2 401 879
- US-A1- 2005 037 058
- US-A1- 2005 037 680

## Description

### FIELD OF THE INVENTION

This invention relates to wound dressings comprising one or more absorbent elements capable of absorbing wound exudates, a non-steroid anti-inflammatory pain-killing agent and a complex of silver ion and a transitional element of group IV of the periodic system of elements.

### BACKGROUND OF THE INVENTION

A wound, ulcer or sore may be defined as an injury to the skin and underlying tissue. Normally, a wound will heal by going through well-characterized healing phases, such as inflammation, granulation, epitheliazation and wound closure. All phases are regulated by a balanced action of proteases and signalling factors (cytokines and growth factors), which are essential to healing.

In a chronic wound, the healing phase has become trapped for reasons not entirely understood. A chronic wound is characterized by a prolonged inflammation, a failure to re-epithelialize and by defective remodelling of the extracellular matrix.

It is generally accepted that bacteria in a wound are detrimental for proper wound healing. Ulcers that are critically colonised with bacteria will often present themselves as painful, malodorous and with increased exudates levels. It is therefore of key importance to obtain and maintain low levels of bacteria and low virulence in the wound in order to avoid stalled wound healing and wound infection. The term critical colonisation has been applied to describe the stage in between normal contamination of a wound and then overt clinical infection, and represents thus an unwanted clinical situation where bacterial imbalance is present in the ulcer.

Wounds are often associated with a chronic, or persistent wound pain. This wound pain is characterised by being present in these wounds all the time, but with varying intensity during the day/night. It is estimated that more than 60% of patients with chronic venous leg ulcers experience chronic pain, i.e. a more persistent type of pain from their ulcer.

Until recently the problem of wound pain has largely been neglected and even ignored. Therefore to date chronic wound pain has been poorly addressed, and research into this subject has been limited. As a consequence of the pain, phenomena like reduced mobility and sleeping problems have a negative impact on the patient's activities of daily living including quality of life.

WO 03/055536 and WO 2004/110511 describe wound dressings capable of delivering an anti-inflammatory pain-killing agent to the wound bed. As an example WO 03/055536 describe wound dressings consisting of a polyurethane foam comprising e.g. ibuprofen as an anti-inflammatory pain-killing agent. WO 2004/110511 describes a dressing comprising a wound-contacting layer consisting of gauze impregnated with a mixture of vaseline, carboxymethyl cellulose and ibuprofen.

One of the great advantages with the foam dressings containing non-steroid anti-inflammatory pain-killing agents (NSAIDs), such as ibuprofen, is that it provides a local pain treatment to the wound and thus eliminates the patient's risk of systemic side effects otherwise often encountered with the oral use of ibuprofen and related NSAIDs.

WO 02/062403 and WO 02/078755 describe medical dressings comprising a complex comprising silver and a transitional element of group IV of the Periodic System of Elements, in particular the silver sodium hydrogen zirconium phosphate complex. These applications describe the preparation of polyurethane foam sheets and hydrogels comprising silver sodium hydrogen zirconium phosphate complex.

Application of a dressing comprising and releasing silver ions to a chronic wound, reduce the number of bacteria by killing them. This will affect the inflammation by reducing it since the bacteria no longer trigger the body's reaction to the infectious agent. The protease concentration in chronic wounds is much higher than in acute wounds. The proteases originate from the body's own cells and from the bacteria, and when present in high concentrations, they degrade important ECM (extra cellular matrix) proteins as well other essential proteins and growth factors. Thus, proteases in high amount delay the wound healing. Silver ions present in a dressing will affect the wound healing positively and the patient's quality of life will increase.

### SUMMARY OF THE INVENTION

The present inventors have, for the first time shown, shown a synergistic pain relieving effect when combing ibuprofen and Silver in a wound dressing. A clinical investigation has proven that the combined pain relieving effect of the pain relieving agent ibuprofen and the antimicrobial agent silver exceeds the effect of these agents used separately.

### DETAILED DESCRIPTION OF THE INVENTION

One aspect of the invention relates to an absorbent polyurethane foam dressing comprising, in the foam, ibuprofen and a complex of silver ion and a transitional element of group IV of the periodic system of elements.

The present invention combines moist wound healing principles with treatment of pain in wounds and treatment of bacterial infection in wounds.

Using a treatment combination that addresses both the bacteria induced pain and the persistent wound pain providers a good possibility for improving patients activities of daily living by reducing the pain as well as initiating ulcer healing that is delayed in critically colonised wounds.

Using a non-steroid anti-inflammatory pain-killing agent in combination with a complex of silver ion and a transitional element of group IV of the periodic system of elements may reduce inflammation in the wound and improve wound healing to a degree which amounts to more than just the additive effect of the pain-killer and the complex of silver ion and a transitional element of group IV of the periodic system of elements.

The absorbent foam dressing will have the ability to exert moist wound healing by absorbing and retaining the wound exudates and thus promoting healing of the wound.

As pain is often encountered in ulcers with critical colonization and in chronic venous leg ulcers, it is important to assess the pain development during the study. Chronic pain may be described as persistent pain (the pain that is always present in varying intensity during the 24 hours a day) and the temporary pain (procedurally related pain at e.g. dressing change). On top of this chronic pain the patient may also experience pain from the critical colonization in the ulcer. This bacterial imbalance in the ulcer is often painful and will untreated lead into clinical infection. By adding silver to the wound, the bacteria - which irritate free nerve endings and liberate nerve irritation enzymes - are killed, and gradually the pain caused by the bacterial infection will decrease.

The present inventors have realised that a dressing comprising a painkiller is rarely used on painful critically colonized wounds. In the dinic, the painkiller is not expected to deal with the infection, and may even camouflage the infection problems. The result is that many patients do not receive adequate treatment for the pain in their wounds. The present invention provide a possibility to treat e.g. painful critically colonized wounds to relief the patient from pain.

The wound pain may be addressed from two angles: Removing the bacteria with the silver treats the colonization, and any residual persistent wound pain is alleviated by the ibuprofen. Relief of the persistent wound pain through a painkiller is not expected to be able to relieve the pain from the infection in the wound, as these two types of pain are induced by different means. But it has surprisingly been shown that the dressing of the present invention is capable of handling the infection problems as well as both the persistent wound pain and the pain from the infection. It is contemplated that the synergistic effect is caused by the attack on both pain-generating sources.

One major disadvantage of a silver containing dressing is that it may cause pain when applied to the wound. The sense of pain is dependent of the exudates level, but can be sensed immediately and can persist in several minutes. On the other hand, the silver ions reduce infection, and thus reduce the pain in the wound caused by the infection. A further observation was that this mild temporary pain was also alleviated by the ibuprofen presence.

Thus, whereas pain reduction by treatment with silver ions includes an initial rise in pain followed later on by a decrease when the infection is reduced, pain reduction by treatment with ibuprofen is more instant as the compound will provide pain relief as soon as it is released to the wound.

The leukocytes may already be in a stressful inflamed environment and the silver ions may increase the sensitivity of the cells causing them into a hyper inflamed state. However, addition of ibuprofen as an anti-inflammatory agent diminishes the inflammation considerably and then has an improved effect on the wound healing.

Even more surprisingly, a synergistic effect is achieved with respect to the pain relieving properties.

In one embodiment of the invention both the non-steroid anti-inflammatory pain-killing agent and the complex of silver ion and a transitional element of group IV of the periodic system of elements is contained in one single absorbent element capable of absorbing wound exudates. Typically, the absorbent element is a layer of polyurethane foam containing a mixture of non-steroid anti-inflammatory pain-killing agent and the complex of silver ion and a transitional element of group IV of the periodic system of elements homogenously distributed in the polyurethane foam.

The non-steroid anti-inflammatory pain-killing agent and the complex of silver ion and a transitional element of group IV of the periodic system of elements may also be contained in two separate absorbing elements capable of absorbing wound exudates. As an example, the dressing comprises two layers of polyurethane foam where one foam layer, comprises the non-steroid anti-inflammatory pain-killing agent homogenously distributed therein and the other foam layer comprises the complex of silver ion and a transitional element of group IV of the periodic system of elements homogenously distributed therein.

According to an alternative embodiment of the invention, the dressing comprises a first and second absorbent element capable of absorbing wound exudates, said first and second absorbent element comprising a non-steroid anti-inflammatory pain-killing agent and a complex of silver ion and a transitional element of group IV of the periodic system of elements, where the amount of non-steroid anti-inflammatory pain-killing agent is higher in said first absorbent element than in said second absorbent element, and the amount of the complex of silver ion and a transitional element of group IV of the periodic system of elements is higher in said second absorbent element than in said first absorbent element. Typically, a device according to this embodiment of the invention comprises two layers of polyurethane foam on top of each other.

In yet another embodiment of the invention the dressing comprises a first and second absorbent element where the first element is a centre circular element with a diameter from 5 to 10 cm, and the second element is a ring around the first element. Each element may comprise one or more of the pharmaceutically active agents. In one embodiment the antibacterial compound comprising silver is contained in the absorbent centre circular element and the anti-inflammatory painkiller is contained in the absorbent ring element surrounding the centre circular element.

The absorbent elements capable of absorbing wound fluid may in principle be placed anywhere in the dressing.

Preferably, the absorbent element capable of absorbing wound exudates is in the form of one layer, or two layers on top of each other.

The absorbent element capable of absorbing wound exudates may be any absorbent element known to be useful for absorption of wound exudates. Wound exudates may appear in different textures, dependent on the stage and character of the wound. Typically it is an aqueous, protein containing fluid and having a viscosity ranging from water-like to highly viscous (slough).

The polyurethane foam of the dressing of the present invention may be substituted or combined with absorbing materials such as other absorbent foams, fibers, hydrogel or paste, hydrogel sheet or a separate element in the form of hydrocolloids or alginates, or a carrier comprising hydrocolloids or alginates distributed in the carrier, e.g. a hydrocolloid adhesive.

Being absorbent is defined by the ability of absorbing liquids, suspensions, moisture, exudate, body fluid, pus or the like. A material is defined as absorbent when it is capable of absorbing at least 0.1 times its own weight.

The foam dressing of the present invention is preferably capable of absorbing 0.1 - 60 times its own weight, more preferred 0.5 - 40 times its own weight, even more preferred 5 - 20 times its own weight and most preferred 8 - 15 times its own weight.

Preferably, the polyurethane foam of the present invention is capable of absorbing wound exudates.

According to one preferred embodiment of the invention, the dressing consists of one foam layers or two foam layers on top of each other.

A skin friendly adhesive may be present on the wound contact surface of the dressing according to the invention. In one embodiment the adhesive is present directly on the surface of the foam layer(s) or around the absorbent element, see e.g. US 2005113733 and WO 99/61077.

In one embodiment of the invention one of the active substances may be present in the adhesive instead of the absorbing element.

A release liner may protect the adhesive surface.

A dressing of the invention may comprise two foam layers with different content of therapeutically active ingredients may comprise a removable top film on both sides of the dressing. Before use, the removable top film is removed from the surface of the dressing, which is to be placed towards the wound surface.

The dressing according to the invention may also comprise a first layer of an absorbent element capable of absorbing wound exudates and comprising a non-steroid anti-inflammatory pain-killing agent and a second layer comprising a complex of silver ion and a transitional element of group IV of the periodic system of elements. Typically, the first layer of an absorbent element is an absorbent foam comprising a non-steroid anti-inflammatory pain-killing agent and the second layer is a gauze or non-woven impregnated with an complex of silver ion and a transitional element of group IV of the periodic system of elements or an adhesive layer comprising a complex of silver ion and a transitional element of group IV of the periodic system of elements. The second layer is typically used as the surface contacting the wound and the area around the wound.

According to another embodiment of the invention, the dressing comprises a first layer of an absorbent element capable of absorbing wound fluids and comprising a complex of silver ion and a transitional element of group IV of the periodic system of elements and a second layer comprising a non-steroid anti-inflammatory pain-killing agent. Typically, the first layer of an absorbent element is a an absorbent foam comprising a complex of silver ion and a transitional element of group IV of the periodic system of elements and the second layer is a gauze or non-woven impregnated with a non-steroid anti-inflammatory pain-killing agent or an adhesive layer comprising non-steroid anti-inflammatory pain-killing agent. The second layer is typically used as the surface contacting the wound and the area around the wound.

The adhesive layer comprising a pharmaceutically active ingredient is suitably a hydrocolloid adhesive.

The ibuprofen of the dressing of the present invention may be substituted by another non-steroid anti-inflammatory pain-killing agent selected from the following chemical groups: Phenylpropionic acids, Phenylacetic acids, Indoleacetic acids, Pyrroleacetic acids, N-Phenylacetic acids, Salicylates, Benzeneacetic acids, Enolic acids, Phenols, Non-acids or Coxibs.

Preferably it could be compounds such as Naproxen, Ketoprofen, Fenoprofen, Flurbiprofen Dexibuprofen, Tiaprofenic acid, Diclofenac, Alclofenac, Fenclofenac, Etodolac, Aceclofenac, Sulindac, Indomethacin, Ketorolac, Tolmetin, Mefenamic acid, Acetyl salicylic acid (Aspirin), Salicylic acid, Diffunisal,Phenylbutazone, Piroxicam, Tenooxicam, Meloxicam or Lomoxicam, Aminopyrene, antipyrene, Acetaminophen, Phenacetin, Nabumeton, Coxib, Celecoxib or Rofecoxib, including any known pro-drug or derivative thereof.

As described in WO 03/055536, the amount of non-steroid anti-inflammatory pain-killing agent in the dressing is typically under the daily oral dose of the compound, i.e. below 200 mg for Naproxen, below 100 mg for Ketoprofen, below 10 mg for Piroxicam, below 1200 mg for lbuprofen, below 200 mg for Celecoxib, below 2 g for acetyl salicylic acid, below 150 mg for Acetaminophen and below 150 mg for Diclofenac. More preferred the amount of non-steroid anti-inflammatory pain-killing agent is below 75 % w/w of the above mentioned doses, more preferred below 50 % w/w of the above mentioned doses, still more preferred below 25 % w/w of the above mentioned doses, even more preferred below 10 or 5 % w/w of the above mentioned doses.

The amount of ibuprofen in the dressing of the present invention is preferably 0.1-2.5 mg/cm² dressing, more preferred 0.2-1.2 mg/cm², and even more preferred 0.3-0.75 mg/cm² dressing and where the total amount of ibuprofen in the dressing does not exceed 1200 mg.

By a transitional element of group IV of the periodic system of elements is meant titanium, zirconium or hafnium. The complex of silver ions and a transitional element of group IV of the periodic system of elements is preferably a silver sodium hydrogen zirconium phosphate complex.

Silver sodium hydrogen zirconium phosphate complex marketed by Milliken under the trademark Alphasan. Alphasan is available in three grades: Alphasan RC 2000 (comprising 10% silver ion), Alphasan 5000 (comprising 3.8% silver ion) and Alphasan RC 7000 (comprising 3.1 % silver ion and 69% zinc oxide).

The amount of the complex of silver ion and the transitional element of group IV of the periodic system of elements in the dressing is typically in the range from 0.01 to 30 mg silver ion/ cm² dressing when calculated as the amount of silver ions, more preferred silver ions is 0.1 - 8 mg/cm² dressing, and even more preferred 0.5 - 1.75 mg/cm² dressing.

The non-steroid anti-inflammatory agent may be incorporated into the dressing as particles, for example as coated particles. The particles may be incorporated into the absorbent element, or impregnated on a layer of gauze or non-woven, or incorporated into an adhesive used on the wound contact surface.

The non-steroid anti-inflammatory pain-killing agent and the complex of silver ion and a transitional element of group IV of the periodic system of elements may be incorporated into the absorbent element after formation of the absorbent element. However, preferably the non-steroid anti-inflammatory pain-killing agent and the complex of silver ion and the transitional element of group IV of the periodic system of elements is incorporated in connection with the preparation of the absorbent element, i.e. during foaming of the foam or preparation of the hydrogel, see for example WO 02/062403 and WO 03/055536.

The complex of silver ion and the transitional element of group IV of the periodic system of elements in the dressing of the present invention may be substituted by another antibacterial agent comprising silver ions. Such agent may be selected from silver sulphadiazide, silver nitrate, silver acetate, silver lactate, silver sulphate, silver sodium thiosulphate, or silver chloride, and zeolites comprising silver.

The release of non-steroid anti-inflammatory pain-killing agent and the complex of silver ion and the transitional element of group IV of the periodic system of elements is controlled by the amount of wound exudates absorbed and retained in the dressing. The pharmaceutical activity of the non-steroid anti-inflammatory pain-killing agent and the complex of silver ion and a transitional element of group IV of the periodic system of elements may be further delayed if desired, by coating the non-steroid anti-inflammatory pain-killing agent and/or the complex of silver ion and a transitional element of group IV of the periodic system of elements.

In this embodiment of the invention the non-steroid anti-inflammatory pain-killing agent and/or the complex of silver ion and a transitional element of group IV of the periodic system of elements may be in the form of coated particles with controlled release properties. The coating may be any suitable coating known in the art of release systems providing the particles with the desired release properties. An example may be particles of non-steroid anti-inflammatory pain-killing agent coated with a polymers such as polyvinylpyrrolidone (PVP), copolymer of vinylpyrrolidone and vinylacetate, carboxymethyl cellulose (CMC), hydroxyethyl cellulose(HEC), hydroxypropyl cellulose (HPC), ethyl cellulose (EC), cellulose acetate phthalate (CAP), copolymers of methacrylic acid methacrylic alkylester, cross-linked polyacrylic acid, PLG (lactide glycolide), polymers of glycerol and palmitostearic acid, polyvinyl alcohol (PVA), Shellac, microcrystalline cellulose (MCC), cellulose acetate phthalate (CAP), polyvinyl acetate phthalate(PVAP), cellulose acetate trimellitate (CAT), hydroxypropyl methylcellulose phthalate (HPMCP), polyurethanes, gelatine or combinations thereof.

The non-steroid anti-inflammatory pain-killing agent and the complex of silver ion and a transitional element of group IV of the periodic system of elements is typically comprised in the absorbent element(s) so that wound exudates is easily brought into contact with the non-steroid anti-inflammatory pain-killing agent and the complex of silver ion and a transitional element of group IV of the periodic system of elements, thereby allowing release to the wound.

Such an absorbing element or combination of absorbing elements comprising the active substances may in one embodiment constitute a wound dressing of the invention. Thus, a broad aspect of the invention relates to an absorbent wound dressing comprising an absorbent element, a pain killing agent and a complex of silver ions with a transitional element of group IV of the periodic system of elements.

In such case, the absorbing element may thus in itself show adhesive properties or it may not show adhesive properties and it will then typically be secured to the desired site using conventional means such as a cover dressing.

The dressing of the invention may comprise a skin-contacting surface comprising an area showing a skin friendly adhesive.

Such a dressing may also comprise a substantially water-impervious layer or film as a backing on the surface facing away from the wound.

The adhesive may be any skin-friendly adhesive known per se, e.g. an adhesive comprising hydrocolloids or other moisture absorbing constituents such as the adhesives disclosed in US patent No. 4,231,369 and in US patent No. 4,367,732 comprising hydrocolloids. A dressing comprising a separate absorbing element may e.g. be of the type disclosed in US Patent No. 5,051,259 or 5,714,225.

A water impervious layer or film may be of any suitable material known per se for use in the preparation of wound dressings e.g. a foam, a non-woven layer or a polyurethane, polyethylene, polyester or polyamide film. A suitable material for use as a water impervious film is a polyurethane such as the low friction film material is disclosed in US patent No. 5,643,187.

In another embodiment of the invention the wound dressing may be a wound cavity filler, e.g. in the form of fibres, gel or hydrogel, foam or powder.

### EXPERIMENTAL PART

### Materials and Methods

Hypol 2002, a polyurethane prepolymer from Dow Chemical.
Pluronic 6200, a PO-PE block copolymer defoamer and surfactant from BASF
Water, distilled water from internal laboratory supply.
Alphasan 2000, silver sodium hydrogen zirconium phosphate available under the Tradename Alphasan® from Milliken Chemical.
Ibuprofen, either USP of Ph.Eur Quality

### EXAMPLE 1: Preparation of a foam dressing comprising Ibuprofen

A polyurethane foam may be prepared in the following way: 100 parts w/w Hypol2002 (Dow Chemical Company) are mixed with 1 part w/w Pluronic 6200 (BASF), 100 parts w/w of water and 1 part w/w Ibuprofen. The materials are mixed together for approximately 15 seconds. The liquid is poured into a mould and allowed to react for 10 minutes. The resulting foam sheet is dried in an oven at 70°C for 30 minutes, and cut into 20 x 20 cm dressings with a thickness of 4,4 mm. The dressing may further be sterilized using gamma radiation.

### EXAMPLE 2: Preparation of a foam dressing comprising silver sodium hydrogen zirconium phosphate complex

A polyurethane foam sheet may be produced by mixing Hypol 2002 (20 grams), Pluronic 6200 (0.2 grams), water (20 grams), silver sodium hydrogen zirconium phosphate (3 grams) by first mixing the water, silver compound and Pluronic and then adding this mixture to the Hypol during mixing. While the mixture still is fluid it is transformed into thin layer by pouring the mixture onto a glass plate, placing a siliconised release paper on the mixture and adjusting the thickness to 2 mm using guiding bars and a doctor roll, allowing the mixture to foam for several minutes. When the material is foamed, the foam sheet is dried in a dry air oven at 130°C. The final foamed sheet have a thickness of 4.5 mm and a content of silver of 9200 mg per square meter of foam (0.92 mg silver /cm²).

A foam dressing consisting of a layer of the foam prepared in example 1 and a layer of the foam in example 2 may be prepared by layering the two foam layers on top of each other and may be used for treatment of infection and pain in a wound. The dressing may be used with either of the two surfaces of the dressing facing the wound, as desired.

### EXAMPLE 3: Preparation of a foam dressing with Ibuprofen and silver sodium hydrogen zirconium sulphate

A polyurethane foam may be prepared in the following way: 100 parts w/w Hypol2002 (Dow Chemical Company) are mixed with 1 part w/w Pluronic 62 (BASF), 100 parts w/w of water, 1 part w/w ibuprofen and 14 part w/w Alphasan (quality comprising 10 % silver ion) The materials are mixed together for approximately 15 seconds. The liquid is poured into a mould and allowed to react for 10 minutes. The resulting foam sheet is dried in an oven at 70°C for 30 minutes, and cut into 10 x 10 cm dressings with a thickness of 4,0 mm. The device may further be sterilized using gamma radiation.

Analogously polyurethane foam dressings comprising Naproxen, Ketoprofen, Piroxicam, Celcoxib, acetylsalicylic acid, Indomethacin, Acetaminophen, Acetylsalicylic arid, diclofenac and/or Mefenamic acid can be prepared using 0.1 part w/w Naproxen, 0.06 part w/w Ketoprofen, 0.04 part w/w Piroxicam, 0.1 part w/w Celecoxib, .5 part w/w acetylsalicylic acid, 0.05 part w/w Indomethacin, 1.5 part w/w acetaminophen, 0.05 part w/w Diclofenac, 0.5 part w/w Mefenamic instead of 1 part w/w of Ibuprofen.

### EXAMPLE 4: Detection of pain relief - a clinical investigation

The investigation was designed as an open, randomized comparative study. The study population consisted of 3 x 3 subjects that were recruited from outpatient clinics at the participating centers. The treatment period per patient was 26±2 days of which the first 5 days mainly concerned pain assessments and the remaining period was concerning the rest of the endpoints e.g. determining the levels of inflammation markers.

The test samples were prepared as Biatain foam dressings, a polyurethane foam dressing marketed by Coloplast A/S. The dressing were produced in three samples, with ibuprofen and/or silver complex incorporated:
BiatainlbuAg is the dressing of the present invention. It is a non-adhesive polyurethane foam dressing with an elastic semi permeable backing film, the dressing comprising ibuprofen and a silver sodium hydrogen zirconium phosphate complex distributed homogenously in the foam.
Biatain-Ibu is a non-adhesive polyurethane foam dressing with an elastic semi permeable backing film and ibuprofen distributed homogenously in the foam.
Biatain-Ag is a non-adhesive polyurethane foam dressing with an elastic semi permeable backing film and silver sodium hydrogen zirconium phosphate complex distributed homogenously in the foam.

The treatment period per subject consisted of 26 (±2 days). The first 3 visits were predefined to be on day 1, 3 and 5 with no margin allowed. The 3 later visits were scheduled to day 12, 19 and 26, all three with a margin on ±2 days.

The patients recorded their perception of pain in the test period, and the results are shown in Table 1 and Table 2.

A patient's experience of wound pain is complex and is influenced by a wide range of factors unique to them. Thus, when subjective experience of pain is reported, it is important to consider the individual's own "pain experience"-starting point at the beginning of the study to obtain the true progress. The progress of pain experience from the first day of treatment to the end is reported using an 11-point numeric Box Scale (NBS), where 10 is severe pain, and 0 is no pain. The progress is evaluated in absolute values, since a change of e.g. 1 using the NBS will be experienced equally for the patients, no matters of the starting point is 8 and is reduced to 7, or 4 and reduced to 3.

Table 1 shows the reduction in pain over the first 5 days, while Table 2 shows the reduction of pain over the whole period (26 days).

**Table 1 the reduction in pain over the first 5 days**

| Dressing | Biatain-Ag | | | Biatain-Ibu | | | Biatain-IbuAg | | |
|---|---|---|---|---|---|---|---|---|---|
| Patient | 101 | 106 | 202 | 102 | 105 | 203 | 103 | 107 | 201 |
| Day 1 | 10 | 4 | 4 | 3 | 6 | 3 | 10 | 7 | 8 |
| Day 5 | 9 | 3 | 2 | 1 | 2 | 1 | 4 | 1 | 0 |
| Pain reduction | 1 | 1 | 2 | 2 | 4 | 2 | 6 | 6 | 8 |
| Average pain reduction | 1,3 | | | 2,7 | | | 6,7 | | |

**Table 2 the reduction of pain over the whole period (26 days)**

| Dressing | Biatain-Ag | | | Biatain-lbu | | | Biatain -lbuAg | | |
|---|---|---|---|---|---|---|---|---|---|
| Patient | 101 | 106 | 202 | 102 | 105 | 203 | 103 | 107 | 201 |
| Day 1 | 10 | 4 | 4 | 3 | 6 | 3 | 10 | 7 | 8 |
| Day 26 | 9 | 1 | 0 | 0 | 4 | 0 | 1 | 0 | 0 |
| Pain reduction | 1 | 3 | 4 | 3 | 2 | 3 | 9 | 7 | 8 |
| Average pain reduction | 2,7 | | | 2,7 | | | 8,0 | | |

As can be seen from the Tables, the reduction in pain when using the combined dressing of the invention is considerably higher than the sum of the reductions of the Biatain-lbu and the Biatain-Ag dressings, thus indicating a clear synergistic effect of the combined dressing. The effect is clear both on day 5 and on day 26.

### EXAMPLE 5 Determination of the content of inflammation markers in the wound exudates

Critical colonization is clinically associated with reduced wound healing, increased exudates levels, foul odor and increased pain. The content of silver in the dressing should diminish the critical colonization in the wound and by that restore a proper wound healing. Therefore inflammatory markers were measured as well as the content of silver in the wound exudates and the area of the ulcer was calculated throughout the investigation period.

In order to determine the content of inflammation markers i.e. interleukins (IL) -1α, IL-6, and IL-8 in wound exudates, ELISA technique was performed. The wound fluid was collected using filter papers and the proteins were eluted in 1 ml ice cold PBS. The samples were taken from the patients of Example 4.

The content of IL-1α in the eluted samples was determined in a capture ELISA described in the protocol of R&D Systems. In brief, the eluted wound fluid samples were diluted appropriately, the monoclonal anti-human IL-1α antibody MAB200 was diluted to 2 µg/ml per well and the biotinylated antibody BAF200 was diluted to 12.5 ng/ml per well (R&D systems).

The content of IL-6 in the eluted samples was determined in a capture ELISA described in the protocol of R&D Systems. In brief, the eluted wound fluid samples were diluted appropriately, the monoclonal anti-human IL-6 antibody MAB206 was diluted to 2 µg/ml per well and the biotinylated antibody BAF206 was diluted to 200 ng/ml per well (R&D systems).

The content of IL-8 in the eluted samples was determined in a capture ELISA described in the protocol of R&D Systems. In brief, the eluted wound fluid samples were diluted appropriately, the monoclonal anti-human antibody IL-8 MAB208 was diluted to 0.5 µg/ml per well and the biotinylated antibody BAF208 was diluted to 20 ng/ml per well (R&D systems).

Due to day-to-day and day/night variation of the level of interleukins and cytokines (inflammation markers) and exudate level, it is very difficult to obtain a clear picture of the effect of silver ions, ibuprofen or the combination of the ibuprofen and silver ions on these levels. A decline of the level of the inflammation markers after treatment with Biatain-lbu (due to the anti-inflammatory effect of ibuprofen) and also - in some lower degree - with the Biatain-Ag treatment (due to reduced/eliminated bacteria amount, the inflammation would be reduced, since the bacteria no longer trigger the body's reaction to the infectious agent) would have been expected. However, this was not the case for the Biatain-Ag or the Biatain-lbu treatment, as can be seen from Table 3.

Surprisingly the data of the combination product of the present invention showed a decline of the inflammation markers for all patients, suggesting that ibuprofen together with silver is able to reduce the inflammation.

The results of the test are shown in Table 3. For each patient is shown starting point (day 1) and end point (day 26). The development in the amount of the markers is shown with arrows, a decrease is shown with a downward arrow, and an increase is shown with an upward arrow. Status quo is shown with a sideward arrow. As can be seen from the Table, the values of the dressing of the invention, Biatain-lbuAg all show a decrease in the content of inflammatory markers, while the Biatain-Ag and the Biatian-lbu show both increase and decrease in the concentrations.

| Treatment | Patient No. | IL1-a in wound fluid [ng/ml] | | IL-6 in wound fluid [ng/ml] | | IL-8 in wound fluid [ng/ml] | |
|---|---|---|---|---|---|---|---|
| Biatain-Ag | 101 | 0.54 | ↓ | 0.13 | ↓ | 53.64 | ↓ |
| | | n.d. | | n.d. | | 0.21 | |
| Biatain-Ag | 106 | 0.09 | → | 0.02 | ↑ | 10.26 | ↑ |
| | | 0.09 | | 0.06 | | 12.23 | |
| Biatain-Ag | 202 | 0.02 | ↑ | 0.52 | ↓ | 7.07 | ↑ |
| | | 0.09 | | 0.07 | | 12.86 | |
| Biatain-lbu | 102 | 0.02 | ↑ | n.d. | n.a. | 5.76 | ↓ |
| | | 0.03 | | n.d. | | 3.76 | |
| Biatain-Ibu | 105 | 0.06 | ↑ | 0.14 | ↓ | 11.75 | ↓ |
| | | 0.09 | | 0.06 | | 5.11 | |
| Biatain-Ibu | 203 | 0.01 | ↑ | 0.05 | ↑ | 1.25 | ↑ |
| | | 0.17 | | 0.56 | | 12.83 | |
| Biatain-lbuAg | 103 | 0.03 | ↓ | 0.07 | ↓ | 15.83 | ↓ |
| | | 0.01 | | 0.01 | | 6.61 | |
| Biatain-lbuAg | 107 | 0.05 | ↓ | 0.03 | ↓ | 5.42 | ↓ |
| | | 0.01 | | n.d. | | 2.91 | |
| Biatain-IbuAg | 201 | 0.03 | ↓ | 0.20 | ↓ | 1.40 | ↓ |
| | | * | | * | | * | |

| | | | | | | | |
|---|---|---|---|---|---|---|---|
| * Wound was healed, and thus no exudate was obtainable. n.d. = not detectable, under the value of detection level: IL1-a= 60 pg/ml, IL-6=75pg/ml, IL-8=63 pg/ml | | | | | | | |

### EXAMPLE 6 Release of Ag+ and Ibuprofen

The release of silver ions and Ibuprofen from the three dressing types, Biatain-Ag, Biatian-Ibu and Biatain-lbuAg was determined.

The release was measured in a release set up (in release model simulating highly exuding wounds) for ibuprofen and silver ions. Franz diffusion cells (Permegear, PA, US) were used, with a volume of 15 ml and a diameter of 2.2 cm. The tested foams were placed on a net between the donor and receptor chamber and covered with an impermeable film. Release of ibuprofen and/or silver from the foams to the receptor media (phosphate buffer pH 7,4) was followed in 7 days. Samples were collected after app. 5, 10, 15, 20, 32, 44, 56, 68, 80, 92, 104, 116, 128, 140, 152, 164 hours and analyzed for ibuprofen/silver using HPLC and AAS, respectively.

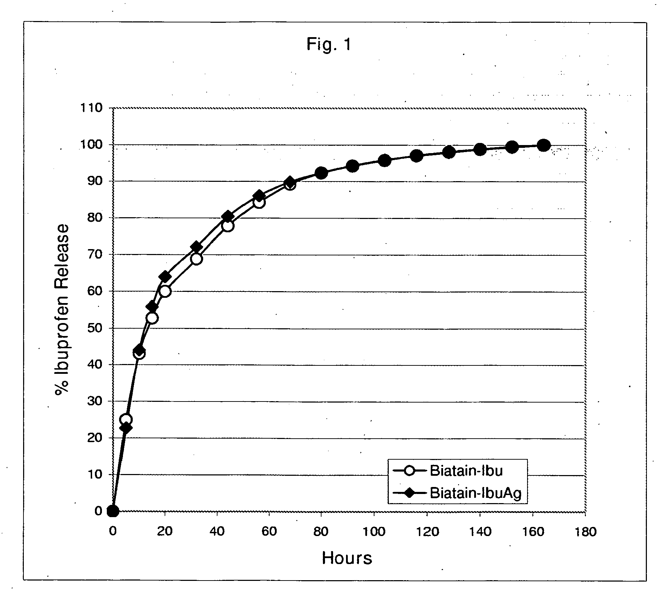

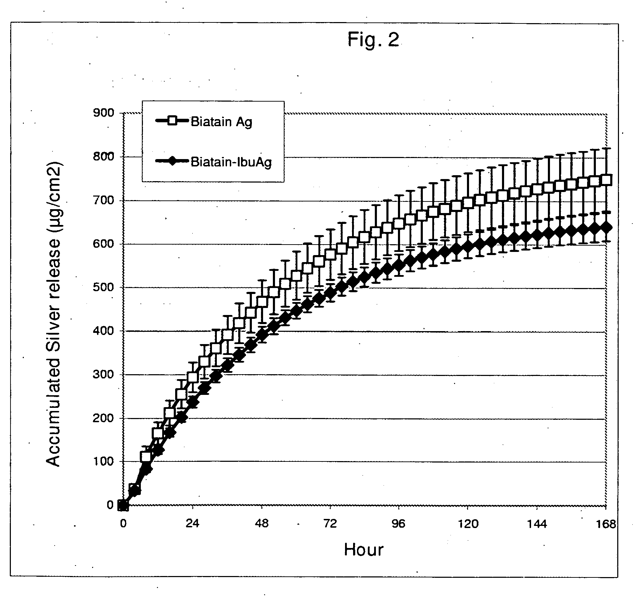

The release profiles for ibuprofen of Biatain-lbu compared to Biatain-lbuAg are shown in Figure 1. The release profiles of silver ions from Biatain-Ag and Biatain-lbuAg are shown in Figure 2.

## Claims

1. An absorbent polyurethane foam dressing comprising, in the foam, ibuprofen and a complex of silver ions with a transitional element of group IV of the periodic system of elements.

2. A dressing according to claim 1 wherein a mixture of ibuprofen and the complex of silver ion and a transitional element of group IV of the periodic system of elements is homogenously distributed in the polyurethane foam.

3. The wound dressing according to any of the preceding claims wherein the complex of silver ion and a transitional element of group IV of the periodic system of elements is silver sodium hydrogen zirconium phosphate complex.

4. The wound dressing according to any of the preceding claims wherein the amount of the complex of silver ion and a transitional element of group IV of the periodic system of elements corresponds to a silver ion amount of 0.01-30 mg silver/cm² in the dressing.

5. The wound dressing according to any of the preceding claims wherein the amount of ibuprofen is preferably 0.1 - 2.5 mg/cm² dressing, more 0.2 -1.2 mg/cm² and where the total amount of ibuprofen in the dressing does not exceed 1200 mg.

6. A dressing according to any of the preceding claims wherein the dressing comprises a water impervious but vapor permeable backing layer.

## Patentansprüche

1. Absorbierender Polyurethanschaum-Verband, der im Schaum Ibuprofen und einen Komplex von Silberionen mit einem Übergangselement der Gruppe IV des Periodischen Systems der Elemente enthält.

2. Verband nach Anspruch 1, bei dem ein Gemisch von Ibuprofen und dem Komplex des Silberions mit einem Übergangselement der Gruppe IV des Periodischen Systems der Elemente im Polyurethanschaum homogen verteilt ist.

3. Wundverband nach einem der vorhergehenden Ansprüche, bei dem der Komplex des Silberions mit einem Übergangselement der Gruppe IV des Periodischen Systems der Elemente ein Silber-Natrium-Zirconium-Wasserstoff-Phosphat-Komplex ist.

4. Wundverband nach einem der vorhergehenden Ansprüche, bei dem die Menge des Komplexes des Silberions mit einem Übergangselement der Gruppe IV des Periodischen Systems der Elemente einer Silberionenmenge von 0,01 - 30 mg Silber/cm² im Verband entspricht.

5. Wundverband nach einem der vorhergehenden Ansprüche, bei dem die Menge an Ibuprofen vorzugsweise 0,1 - 2,5 mg/cm² Verband, noch bevorzugter 0,2 - 1,2 mg/cm² beträgt und wobei die Gesamtmenge an Ibuprofen im Verband 1200 mg nicht übersteigt.

6. Verband nach einem der vorhergehenden Ansprüche, wobei der Verband eine wasserundurchlässige, jedoch dampfdurchlässige Rückschicht aufweist.

## Revendications

1. Pansement en mousse de polyuréthane absorbante comprenant, dans la mousse, de l'ibuprofène et un complexe d'ions d'argent avec un élément transitionnel du groupe IV de la classification périodique des éléments.

2. Pansement selon la revendication 1, dans lequel un mélange d'ibuprofène et du complexe composé d'ions d'argent et d'un élément transitionnel du groupe IV de la classification périodique des éléments est réparti de manière homogène dans la mousse de polyuréthane.

3. Le pansement pour plaies selon l'une quelconque des revendications précédentes, dans lequel le complexe composé d'ions d'argent et d'un élément transitionnel du groupe IV de la classification périodique des éléments est un complexe d'argent, de sodium, d'hydrogène, de zirconium et de phosphate.

4. Le pansement pour plaies selon l'une quelconque des revendications précédentes, dans lequel la quantité que représente le complexe composé d'ions d'argent avec un élément transitionnel du groupe IV de la classification périodique des éléments correspond à une quantité d'ions d'argent de 0.01 à 30 mg d'argent/cm² dans le pansement.

5. Le pansement pour plaies selon l'une quelconque des revendications précédentes, dans lequel la quantité d'ibuprofène représente de préférence de 0.1 à 2.5 mg/cm² dans le pansement, encore mieux de 0.2 à 1.2 mg/cm², et où la quantité totale d'ibuprofène dans le pansement n'excède pas 1200 mg.

6. Pansement selon l'une quelconque des revendications précédentes, dans lequel le pansement comprend une couche de renfort perméable à la vapeur mais imperméable à l'eau.
